# EUROPEAN PATENT APPLICATION

(11) **EP 4 837 576 A1**
(43) Date of publication of application: **11.11.2026**
(21) Application number: 25736331.7
(22) Date of filing: 06.01.2025
(51) Int. Cl.: A61K 31/496, A61K 31/09, A61K 31/17, A61K 31/4178, A61K 31/12, A61K 31/519, A61K 31/337, A61K 31/472, A61K 31/4184, A61K 31/5377

(54) **COMPOSITION COMPRISING TUBULIN INHIBITOR FOR PREVENTING OR TREATING SKIN DISEASES**

(30) Priority: 05.01.2024 KR 20240001890
(71) Applicant: Cuepeak Bio. Co., Ltd., Daejeon 34141 (KR)
(72) Inventor: HWANG, Joonsung, Daejeon 34141 (KR); KIM, Jeeeune, Daejeon 34141 (KR)
(74) Representative: dompatent
(86) International application number: PCT/KR2025/000257
(87) International publication number: WO 2025/147178

(57) **Abstract**

The present disclosure relates to a pharmaceutical composition comprising a tubulin inhibitor as an active ingredient for preventing or treating skin diseases. In addition, the present disclosure relates to a quasi-drug composition or cosmetic composition comprising a tubulin inhibitor as an active ingredient for preventing or alleviating skin diseases. In addition, the present disclosure relates to a cosmetic composition comprising a tubulin inhibitor as an active ingredient for skin moisturization, whitening, wrinkle alleviation, acne relief, skin barrier strengthening, or skin keratinocyte differentiation. The tubulin inhibitor of the present disclosure increases the expression level of filaggrin in epidermal cells, promotes skin differentiation, inhibits NF-κB signaling induced by TNF-α, and exhibits photo-aging inhibition and wrinkle alleviation effects as well as whitening effects. Accordingly, the tubulin inhibitor of the present disclosure can be utilized as an active ingredient that simultaneously exhibits activities, such as skin moisturization, whitening, wrinkle alleviation, acne relief, skin barrier strengthening, or skin keratinocyte differentiation, along with the prevention, treatment, or alleviation of skin diseases.

## Description

### [Technical Field]

The present disclosure relates to a pharmaceutical composition including a tubulin inhibitor as an active ingredient for preventing or treating skin diseases.

In addition, the present disclosure relates to a quasi-drug composition including a tubulin inhibitor as an active ingredient for preventing or alleviating skin diseases.

In addition, the present disclosure relates to a cosmetic composition including a tubulin inhibitor as an active ingredient for preventing or alleviating skin diseases.

In addition, the present disclosure relates to a cosmetic composition including a tubulin inhibitor as an active ingredient for skin moisturization, whitening, wrinkle alleviation, acne relief, skin barrier strengthening, or skin keratinocyte differentiation.

### [Background Art]

A major function of the skin serves as a barrier for separating an internal environment from an external environment, and to protect against attacks of foreign substances and minimize the loss of moisture and other essential body ingredients to an external space. Filaggrin is particularly important in skin barrier formation, which is due to a fundamental role in the final differentiation of the epidermis and association with skin diseases. The filaggrin was first identified as an important structural protein in 1977, and then found to serve to aggregate and compress keratin intermediate filaments, which was named as an abbreviation for filament-aggregating protein. This protein is synthesized from a large precursor protein, profilaggrin, which is a major component of keratohyalin granules in the granulosum layer of the epidermis.

The main element of the skin barrier is the stratum corneum. This stratum corneum is a result product of differentiation of keratinocytes while migrating from the basal layer to the granulosum layer. While these cells mature, the cells express a variety of structural proteins.

The filaggrin is continuously processed by various proteases. Such a proteolysis process releases hygroscopic amino acids and their derivatives, which serves to form a natural moisturizing factor (NMF) to retain moisture in the stratum corneum.

That is, the filaggrin is converted from a precursor protein, profilaggrin to an active filaggrin monomer, and aggregates and compresses keratin filaments to flatten keratinocytes and then forms the stratum corneum, and the natural moisturizing factor (NMF) produced during the filaggrin processing process contributes to maintaining moisture in the skin and controlling acidic pH.

In addition, the filaggrin binds intermediate keratin filaments to a structural protein of the cornified epithelium (CE), so that the keratinocytes have strong mechanical and chemical resistance. The filaggrin also contributes to the formation of the extracellular lipid matrix of the stratum corneum together with lipids released from lamellar bodies.

In particular, filaggrin degradation products such as urocanic acid (UCA) and pyrroliodone-5-carboxylic acid (PCA) maintain skin moisture, maintain acidic pH, and protect the skin from UV radiation. In addition, the UCA plays an important role in antimicrobial activity and immune regulation.

### [Disclosure]

### [Technical Problem]

Accordingly, the present inventors confirmed that a tubulin inhibitor increased the expression of filaggrin in epidermal cells and promoted the differentiation of the epidermal cells. In addition, more surprisingly, the present inventors confirmed that the tubulin inhibitor inhibited inflammatory signals, inhibited photoaging, inhibited collagen degradation mechanisms, and even had a whitening effect, and then completed the present disclosure.

Therefore, an object of the present disclosure is to provide a pharmaceutical composition and a quasi-drug composition for preventing or treating skin diseases. Another object of the present disclosure is to provide a cosmetic composition for preventing or alleviating skin diseases and a cosmetic composition for skin moisturization, whitening, wrinkle alleviation, acne relief, skin barrier strengthening, or skin keratinocyte differentiation.

### [Technical Solution]

In order to achieve the object, an aspect of the present disclosure provides a pharmaceutical composition including a tubulin inhibitor as an active ingredient for preventing or treating skin diseases.

Another aspect of the present disclosure provides a quasi-drug composition and a cosmetic composition including a tubulin inhibitor as an active ingredient for preventing or alleviating skin diseases.

Yet another aspect of the present disclosure provides a cosmetic composition including a tubulin inhibitor as an active ingredient for skin moisturization, whitening, wrinkle alleviation, acne relief, skin barrier strengthening, or skin keratinocyte differentiation.

### [Advantageous Effects]

According to the present disclosure, the tubulin inhibitor increases the expression level of filaggrin in epidermal cells, promotes skin differentiation, inhibits NF-κB signaling induced by TNF-α, and exhibits photo-aging inhibition and wrinkle alleviation effects, as well as whitening effects. Accordingly, the tubulin inhibitor of the present disclosure can be utilized as an active ingredient that simultaneously exhibits activities, such as skin moisturization, whitening, wrinkle alleviation, acne relief, skin barrier strengthening, or skin keratinocyte differentiation, along with the prevention, treatment, or alleviation of skin diseases.

### [Description of drawings]

FIG. 1 relates to an effect of a tubulin inhibitor on the expression level of filaggrin in a normal human epidermal keratinocyte (NHEK). FIG. 1 shows a result of increasing the expression level of filaggrin according to treatment of the tubulin inhibitor.
FIG. 2 relates to an effect of a tubulin inhibitor on cell differentiation in a normal human epidermal keratinocyte (NHEK). FIG. 2 shows a result of promoting cell differentiation after treatment of the tubulin inhibitor.
FIG. 3 relates to an experimental result for confirming an inhibitory effect of a tubulin inhibitor on NF-κB signaling. FIG. 3 shows a result of inhibiting NF-κB signaling after treatment of the tubulin inhibitor.
FIG. 4 relates to an effect of a tubulin inhibitor on reduction of the expression levels of MMP-1, MMP-3, and MMP-9 induced by photoaging. FIG. 4 shows a result of inhibiting the expression level of MMP induced by UV by the tubulin inhibitor.
FIG. 5 relates to effects of a tubulin inhibitor on inhibition of tyrosinase activity and whitening. FIG. 5 shows a result of reducing melanin production by inhibiting tyrosinase activity by the tubulin inhibitor.

### [Best Mode]

Hereinafter, the present disclosure will be described in detail.

The present disclosure provides a pharmaceutical composition including a tubulin inhibitor as an active ingredient for preventing or treating skin diseases.

In addition, the present disclosure provides a method for preventing or treating skin diseases, including administering a tubulin inhibitor in a therapeutically effective amount to a subject in need thereof.

The tubulin inhibitor refers to a drug group that regulates or inhibits the function of tubulin protein as a major component of microtubules. The microtubule is a part of the cytoskeleton that plays an important role in cells and is an essential structure for processes such as cell division, cell migration, and intracellular transport.

In the present disclosure, unlike these previous functions, the tubulin inhibitor exhibits the activities in increased expression level of filaggrin in epidermal cells and skin proliferation according to epidermal cell differentiation, inhibits NF-κB signaling induced by TNF-α, exhibits photo-aging inhibition and wrinkle alleviation effects, and exhibits a whitening effect.

In the present disclosure, the tubulin inhibitor is not limited in type, but may be, for example, at least one selected from the group consisting of vinca alkaloid, taxane, cryptophycin 52, halichondrins, dolastatins, hemiasterlins, combretastatin-A4, 2-methoxyestradiol, E7010, plinabuline, epothilone, and discodermolide.

The vinca alkaloid of the present disclosure may preferably be vinblastine, vincristine, vinorelbine, or vinflunine.

In addition, the taxane may be paclitaxel or docetaxel.

The tubulin inhibitor of the present disclosure is a concept including all pharmaceutically acceptable salts, isomers, or derivatives thereof, as well as analogs exhibiting the same or similar effects.

In the present disclosure, the pharmaceutically acceptable salts refer to salts commonly used in the pharmaceutical industry. For example, the pharmaceutically acceptable salts may include inorganic ion salts prepared with calcium, potassium, sodium, magnesium, etc.; inorganic acid salts prepared with hydrochloric acid, nitric acid, phosphoric acid, bromic acid, iodic acid, perchloric acid, sulfuric acid, etc.; organic acid salts prepared with acetic acid, trifluoroacetic acid, citric acid, maleic acid, succinic acid, oxalic acid, benzoic acid, tartaric acid, fumaric acid, mandelic acid, propionic acid, citric acid, lactic acid, glycolic acid, gluconic acid, galacturonic acid, glutamic acid, glutaric acid, glucuronic acid, aspartic acid, ascorbic acid, carboxylic acid, vanillic acid, hydroiodic acid, etc.; sulfonic acid salts prepared with methanesulfonic acid, ethanesulfonic acid, benzenesulfonic acid, p-toluenesulfonic acid, naphthalenesulfonic acid, etc.; amino acid salts made from glycine, arginine, lysine, etc.; and amine salts prepared with trimethylamine, triethylamine, ammonia, pyridine, picoline, etc., but are not limited thereto.

The tubulin inhibitor may be derived from an extract, produced biologically, or synthesized chemically, and is not limited in its origin and preparation method.

In the present disclosure, the skin diseases correspond to a group of diseases caused by structural and functional abnormalities of the skin, and mean a concept of encompassing abnormal conditions occurring in the skin that may be caused by various causes, such as inflammation, infection, allergy, and immune abnormality.

In the present disclosure, the skin diseases may be at least one selected from the group consisting of, for example, atopic dermatitis, skin thermal damage, pruritus, acne, psoriasis, allergic dermatitis, contact dermatitis, exfoliative dermatitis, seborrheic dermatitis, seborrheic scalpitis, lichen planus, rosacea, pigmentation disorder, hypermelanosis, erythema, wounds, ulcers, bedsores, lupus, skin wrinkle-related diseases, and skin diseases caused by photodamage, and the type thereof is not limited.

The skin wrinkle-related disease may include elastic fibrosis, thinning of the skin, skin atrophy, reduction of collagen fibers and elastic fibers, loss of skin elasticity, dryness, wrinkle formation, or premature skin aging.

In addition, the skin disease caused by the photodamage may include lentigines, freckles, hypopigmentation, hyperpigmentation, acute or chronic photodamage caused by UV radiation, or photosensitization.

In addition, the skin diseases of the present disclosure may include squamous cell carcinoma, basal cell carcinoma, benign epithelial tumor, and radiation dermatitis.

In addition, the skin diseases of the present disclosure may include panniculitis, calluses, vitiligo, urticaria, folliculitis, sebaceous keratosis pilaris, eczema, corns, melasma, rashes, athlete's foot, spots, stretch marks, freckles, prickly heat, dry skin, chilblains, boils, pockmarks, scalpitis or psoriatic arthritis.

In addition, the present disclosure may further include a compound selected from the group consisting of calcium, colchicine, tapinarof, fingolimod, and tofacitinib, in addition to the tubulin inhibitor.

The compound selected from the group consisting of calcium, colchicine, tapinarof, fingolimod, and tofacitinib may be used in combination or mixed with the tubulin inhibitor, and may be simultaneously or sequentially administered.

The calcium, colchicine, tapinarof, fingolimod, or tofacitinib corresponds to a concept that includes all of their salts, isomers, or derivatives, and analogs exhibiting the same or similar effects.

In the present disclosure, the tubulin inhibitor may be provided as an active ingredient to the pharmaceutical composition for preventing or treating skin diseases.

The term "prevention" in the present disclosure may include inhibiting the occurrence of diseases.

The term "treatment" in the present disclosure includes inhibition, alleviation, or elimination of the development of diseases.

In the present disclosure, the term "including as the active ingredient" means that the tubulin inhibitor of the present disclosure is added so as to exhibit the effects mentioned in the present disclosure, and includes formulation in various forms by adding various ingredients as accessory ingredients for drug delivery and stabilization.

The pharmaceutical composition may include the tubulin inhibitor in a pharmaceutically effective amount.

In the present disclosure, the term "pharmaceutically effective amount" means an amount sufficient to achieve the efficacy or activity of the active ingredient.

The pharmaceutical composition may further include a pharmaceutically acceptable diluent or carrier. The diluent may be lactose, corn starch, soybean oil, microcrystalline cellulose, mannitol, or a combination thereof. The carrier may be an excipient, a disintegrant, a binder, a lubricant, or a combination thereof. The excipient may be microcrystalline cellulose, lactose, low-substituted hydroxycellulose, or a combination thereof. The disintegrant may be calcium carboxymethylcellulose, sodium starch glycolate, calcium dihydrogen phosphate anhydrous, or a combination thereof. The binder may be polyvinylpyrrolidone, low-substituted hydroxypropyl cellulose, hydroxypropyl cellulose, or a combination thereof. The lubricant may be magnesium stearate, silicon dioxide, talc, or a combination thereof.

The pharmaceutical composition may be prepared into oral or parenteral administration formulations. The oral formulations may include granules, powders, liquids, tablets, capsules, dry syrups, or combinations thereof. The parenteral formulations may include injections, ointments, aerosols, sprays, patches, etc., and the formulations thereof are not limited.

The dose of the pharmaceutical composition according to an embodiment may be administered to mammals for 1 day in an amount of, for example, about 0.0001 to 10,000 mg/kg, 0.0001 to 5,000 mg/kg, 0.0001 to 1,000 mg/kg, 0.0001 to 900 mg/kg, 0.0001 to 800 mg/kg, 0.0001 to 700 mg/kg, 0.0001 to 600 mg/kg, 0.0001 to 500 mg/kg, 0.0001 to 400 mg/kg, 0.0001 to 300 mg/kg, 0.0001 to 200 mg/kg, 1 to 1,000 mg/kg, 1 to 900 mg/kg, 1 to 800 mg/kg, 1 to 700 mg/kg, 1 to 600 mg/kg, 1 to 500 mg/kg, 1 to 450 mg/kg, 1 to 400 mg/kg, 1 to 350 mg/kg, 1 to 300 mg/kg, 1 to 250 mg/kg, 10 to 1,000 mg/kg, 10 to 900 mg/kg, 10 to 800 mg/kg, 10 to 700 mg/kg, 10 to 600 mg/kg, 10 to 500 mg/kg, 10 to 450 mg/kg, 10 to 400 mg/kg, 10 to 350 mg/kg, 10 to 300 mg/kg, 10 to 250 mg/kg, 100 to 1,000 mg/kg, 100 to 900 mg/kg, 100 to 800 mg/kg, 100 to 700 mg/kg, 100 to 600 mg/kg, 100 to 500 mg/kg, 100 to 450 mg/kg, 100 to 400 mg/kg, 100 to 350 mg/kg, 100 to 300 mg/kg, 100 to 250 mg/kg, 200 to 1,000 mg/kg, 200 to 900 mg/kg, 200 to 800 mg/kg, 200 to 700 mg/kg, 200 to 600 mg/kg, 200 to 500 mg/kg, 200 to 450 mg/kg, 200 to 400 mg/kg, 200 to 350 mg/kg, 200 to 300 mg/kg, or 200 to 250 mg/kg, but is not limited thereto. The frequency of administration of the pharmaceutical composition of the present disclosure is not particularly limited, but the pharmaceutical composition may be administered once a day or several times a day by dividing the dose. The dose is not limited to the scope of the present disclosure in any aspect.

The subject to be administered with the pharmaceutical composition provided in the present disclosure may be mammals, including human, dog, cat, horse, cow, pig, goat, rabbit, mouse, rat, etc., or cells, tissues, or culture solutions thereof isolated therefrom. In an example, the subject may be a subject (mammals such as human) or cells, tissues, or culture solutions thereof isolated therefrom in need of prevention, alleviation, and/or treatment of skin diseases or having skin diseases as described above.

The pharmaceutical composition according to an embodiment may include the tubulin inhibitor in an amount of 1 to 80 wt%, 5 to 80 wt%, 5 to 75 wt%, 5 to 70 wt%, 5 to 65 wt%, 50 to 70 wt%, 55 to 65 wt%, 60 to 65 wt%, 10 to 60 wt%, 15 to 60 wt%, 20 to 60 wt%, 1 to 50 wt%, 5 to 50 wt%, 10 to 50 wt%, 15 to 50 wt%, 20 to 50 wt%, 1 to 40 wt%, 5 to 40 wt%, 10 to 40 wt%, 15 to 40 wt%, 20 to 40 wt%, 1 to 30 wt%, 5 to 30 wt%, 10 to 30 wt%, 15 to 30 wt%, 20 to 30 wt%, 1 to 25 wt%, 5 to 25 wt%, 10 to 25 wt%, 15 to 25 wt%, 20 to 25 wt%, or 23 to 25 wt%.

Alternatively, the composition may be a skin quasi-drug composition.

The skin quasi-drug may be cream, gel, ointment, skin emulsifier, skin suspension, transdermal patch, drug-containing bandage, lotion, spray, or a combination thereof, but is not limited thereto.

In the present disclosure, the skin quasi-drug may be appropriately mixed with ingredients commonly used in skin quasi-drugs such as cosmetics or medicines, such as aqueous ingredients, oily ingredients, powder ingredients, alcohols, moisturizers, thickeners, ultraviolet absorbers, whitening agents, preservatives, antioxidants, surfactants, fragrances, colorants, various skin nutrients, metal ion sequestrants, sugars, or combinations thereof, as needed.

In addition, the present disclosure provides a quasi-drug composition including a tubulin inhibitor as an active ingredient for preventing or alleviating skin diseases.

In the present disclosure, the quasi-drug refers to all products other than drugs related to the treatment or prevention of diseases.

In the present disclosure, the quasi-drug refers to all products other than drugs related to the treatment or prevention of diseases. The quasi-drug means products that have a milder effect than drugs among products used for the purpose of diagnosing, treating, alleviating, reducing, managing or preventing diseases of humans or animals. For example, according to the Pharmaceutical Affairs Act, the quasi-drugs are products excluding products used for the purpose of medicines, and may include fiber and rubber products used for the treatment or prevention of diseases of humans or animals, products that have a mild or no direct effect on the human body and are not instruments or machines or similar thereto, sterilizers and insecticides for preventing infectious diseases, etc., but are not limited thereto.

The type or formulation of the quasi-drug composition of the present disclosure is not particularly limited, but may preferably be a disinfectant, a shower foam, a gargle, a wet tissue, a detergent soap, a hand wash, a humidifier filler, a mask, an ointment, a filter filler, or the like.

When the composition of the present disclosure is included in the quasi-drug for the purpose of skin moisturization, the composition may be used as it is or used in combination with other quasi-drug ingredients, and may be used appropriately according to conventional methods. The mixing amount of the active ingredient may be appropriately determined according to the purpose of use, and the quasi-drug composition according to the present disclosure may contain the tubulin inhibitor in an amount of 0.01 to 20 wt% based on the total weight of the composition.

In addition, the present disclosure provides a cosmetic composition including a tubulin inhibitor as an active ingredient for preventing or alleviating skin diseases.

The term "alleviation" in the present disclosure may mean all actions that at least reduce parameters associated with alleviation or treatment of conditions, for example, the degree of symptoms.

The tubulin inhibitor may be included in an amount of 0.0001 to 80 wt%, 0.01 to 70 wt%, 0.01 to 60 wt%, 0.01 to 50 wt%, 0.01 to 40 wt%, 0.01 to 30 wt%, 0.1 to 70 wt%, 0.1 to 60 wt%, 0.1 to 50 wt%, 0.1 to 40 wt%, 0.1 to 30 wt%, 0.5 to 70 wt%, 0.5 to 60 wt%, 0.5 to 50 wt%, 0.5 to 40 wt%, 0.5 to 30 wt%, 1 to 70 wt%, 1 to 60 wt%, 1 to 50 wt%, 1 to 40 wt%, or 1 to 30%, for example, 30 wt% based on the total weight of the cosmetic composition, but is not limited thereto.

The cosmetic composition is not particularly limited to a specific formulation, and the formulation may be appropriately selected depending on the purpose. The cosmetic composition may have, for example, a solubilized formulation, an emulsified formulation, or a dispersed formulation. The cosmetic composition may have a cosmetic formulation of a skin toner, a nourishing toner, a massage cream, a nourishing cream, an essence, a pack, a gel, an ampoule, or a skin adhesive type, but is not limited thereto.

The cosmetic composition may further include ingredients commonly used in the cosmetic composition in addition to the active ingredient described in the present disclosure, and may include, for example, conventional adjuvants and carriers, such as antioxidants, stabilizers, solubilizers, surfactants, dispersants, emulsifiers, preservatives, vitamins, pigments, and fragrances.

In addition, the present disclosure provides a cosmetic composition including a tubulin inhibitor as an active ingredient for skin moisturization, whitening, wrinkle alleviation, acne relief, skin barrier strengthening, or skin keratinocyte differentiation.

In the present disclosure, the term "skin moisturization" may mean all actions that maintain skin moisture or prevent moisture loss.

In the present disclosure, the term "whitening" may mean improving the brightness of skin tone or alleviating pigmentation by inhibiting melanin production.

In the present disclosure, the term "wrinkle alleviation" may mean increasing skin elasticity, reducing the depth of wrinkles, or increasing the smoothness of the skin surface.

In the present disclosure, the term "acne relief" may mean inhibiting inflammation, redness, or excessive sebum production of acne, or promoting healing of acne lesions.

In the present disclosure, the term "skin barrier strengthening" may mean all actions that enhance the functions of the skin barrier, which is located at the outermost part of the skin to prevent moisture and nutrient loss.

In the present disclosure, the term "skin keratinocyte differentiation" may mean promoting a process of maturing keratinocytes to form the stratum corneum, or strengthening the function of the skin barrier.

Yet another aspect of the present disclosure provides a method for preventing, alleviating, or treating skin diseases, including administering an effective amount of the tubulin inhibitor to a subject in need thereof.

The term "effective amount" means an amount effective to exhibit the effects mentioned above.

The subject may be mammals, for example, human, cow, horse, pig, dog, sheep, goat, or cat. The subject may be a subject requiring the effect of preventing, alleviating, or treating skin diseases.

In the present disclosure, the term "administering" is used interchangeably with "introducing" or "implanting", and may mean placement of a composition according to an embodiment into a subject by a method or route that causes at least partial localization of the composition according to an embodiment to a desired site.

The administration may be performed by methods known in the art. The administration method, including the route of administration and the number of administrations, may be appropriately selected by those skilled in the art. For example, the administration may be directly performed to the subject by any means through routes, such as intravenous, intramuscular, oral, transdermal, mucosal, intranasal, intraoral, intratracheal or subcutaneous administration. The administration may be performed systemically or locally. The administration may include application to the skin.

In the present disclosure, the composition according to one specific example may be administered per subject a day in an amount of 0.1 mg to 1,000 mg, for example, 0.1 mg to 500 mg, 0.1 mg to 100 mg, 0.1 mg to 50 mg, 0.1 mg to 25 mg, 1 mg to 1,000 mg, 1 mg to 500 mg, 1 mg to 100 mg, 1 mg to 50 mg, 1 mg to 25 mg, 5 mg to 1,000 mg, 5 mg to 500 mg, 5 mg to 100 mg, 5 mg to 50 mg, 5 mg to 25 mg, 10 mg to 1,000 mg, 10 mg to 500 mg, 10 mg to 100 mg, 10 mg to 50 mg, or 10 mg to 25 mg. However, the dose may be variously prescribed by factors, such as a formulation method, an administration method, the age, weight, sex, and a pathological condition of a patient, diet, an administration time, an administration route, an excretion rate, and response susceptibility, and those skilled in the art may appropriately adjust the dose by considering these factors. The number of administrations may be once a day or twice or more within the range of clinically acceptable side effects, and the administration site may be one or two or more sites, and the total number of administration days may be from 1 to 30 days during once treatment, every day or at intervals of 2 to 5 days. If necessary, the same treatment may be repeated after an appropriate period of time. Even for animals other than humans, the same dose per kg as for humans may be administered, or the dose may be administered in an amount converted the above-described dose by a volume ratio (e.g., average value) of organs (heart, etc.) between target animal and human.

Yet another aspect provides a use of the tubulin inhibitor for use in preparing a composition for preventing, alleviating, or treating skin diseases.

Yet another aspect provides a use of the tubulin inhibitor for use in preparing a drug for preventing, alleviating, or treating skin diseases.

Yet another aspect provides a food composition including a tubulin inhibitor as an active ingredient for preventing or alleviating skin diseases.

The food composition according to an embodiment of the present disclosure may be liquid or solid formulations, and may be tablets, capsules, soft capsules, pills, granules, beverages (drinks), diet bars, chocolate, caramel formulations, or confectionery formulations, and the formulations are not particularly limited. The food composition of the present disclosure may appropriately contain excipients, sugars, flavorings, pigments, fats, proteins, etc. as needed, in addition to the active ingredient.

Duplicated contents will be omitted in consideration of the complexity of the present specification, and terms not defined otherwise herein have the meanings commonly used in the art to which the present disclosure pertains.

### [Modes]

Hereinafter, the present disclosure will be described in detail by Examples. However, the following Examples are just illustrative of the present disclosure, and the contents of the present disclosure are not limited to the following Examples.

### [Experimental Example 1] Strain

A normal human epidermal keratinocyte (NHEK) cell line was a human fetal epidermal cell line and was obtained from ATCC and used. The cell line was cells obtained through primary culture and used to confirm intracellular biochemical changes caused by a tubulin inhibitor under conventional conditions rather than disease conditions.

A human embryonic kidney (HEK) cell line was obtained from ATCC and used. The cell line was used to confirm intracellular biochemical changes caused by the tubulin inhibitor under conventional conditions rather than disease conditions.

### [Experimental Example 2]

### 1.1 Measurement of expression level of filaggrin using measurement of cell-based promoter activity

A normal human epidermal keratinocyte (NHEK) was inserted with a lucifer luminescent gene with a filaggrin promoter using a Fugene4K reagent (Promega), and then after 24 hours, treated with a tubulin inhibitor at a concentration of 1.0 µM. 24 hours after drug treatment, the expression level of filaggrin according to treatment with each tubulin inhibitor was measured using a kit (Promega) of measuring the degree of lucifer luminescence. As a control group, the same volume of DMSO as the treated compound was used.

### 1.2 Observation of cell differentiation measurement using phase-contrast microscope images

A normal human epidermal keratinocyte (NHEK) cell line was treated with each tubulin inhibitor at a concentration of 1.0 µM, and the degree of cell differentiation was observed daily using a phase-contrast microscope.

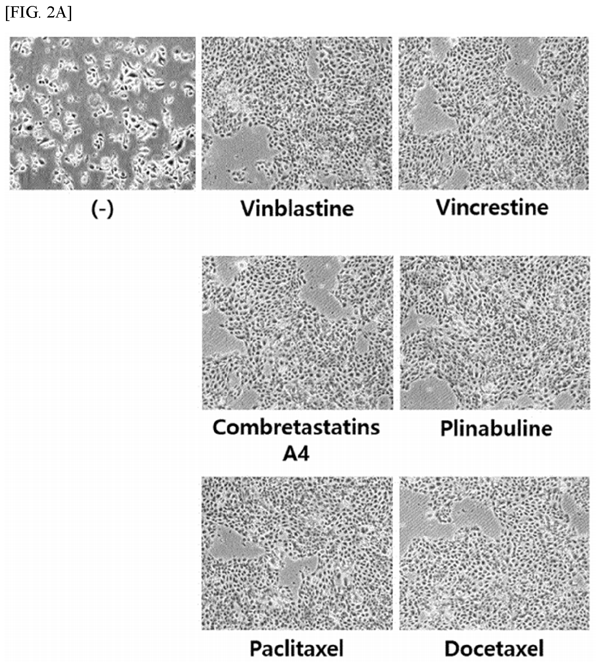

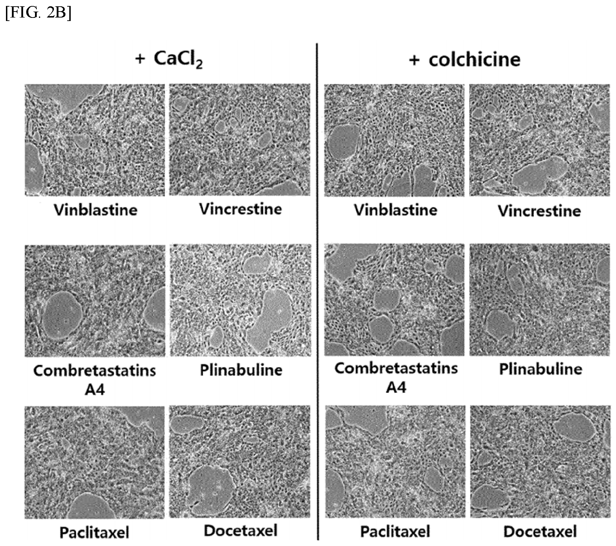

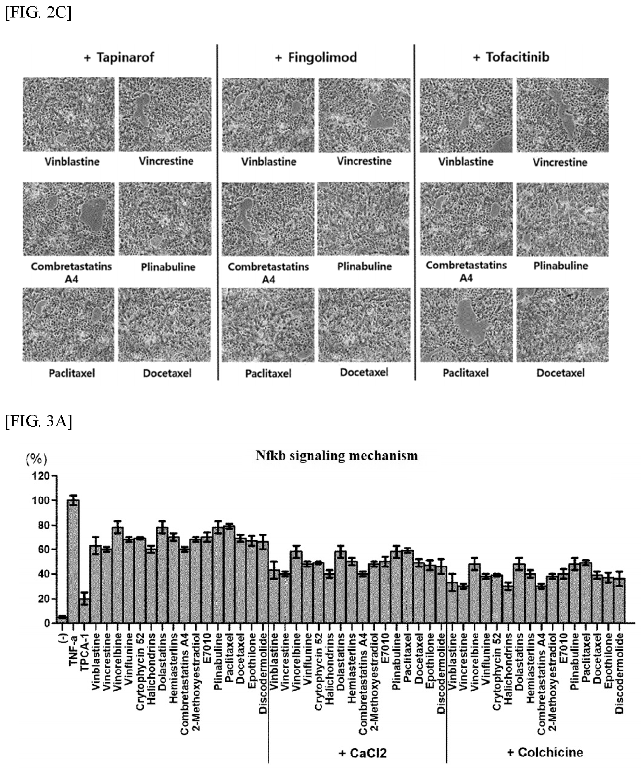

The image was taken with a phase-contrast microscope camera on day 5 of cell differentiation, and the results were shown in FIG. 2.

### [Experimental Example 3] Confirmation of inhibition of inflammatory signaling mechanism using measurement of cell-based promoter activity

NF-κB, a DNA transcription factor, was present in the cytosol and present in a form conjugated with IκB inhibiting NF-κB. The NF-κB was a representative pro-inflammatory cytokine that was involved in innate and acquired immune responses and shown in many inflammatory diseases.

The NF-κB mechanism was a protein family involved in inflammatory response regulation, immune modulation, apoptosis, cell proliferation, epithelial differentiation, etc. and regulated the expression of various genes and formed a central axis of the intracellular signaling system.

A human embryonic kidney cell line (HEK) was inserted with a lucifer luminescent gene having an NFkb promoter using a Fugene4K reagent (Promega), and then treated with TNF-α after 6 hours to induce inflammation and NfKb signaling. After 18 hours, each tubulin inhibitor was treated at a concentration of 1.0 µM.

24 hours after drug treatment, an inhibitory effect on an NfkB signaling mechanism according to treatment with each tubulin inhibitor was measured using a kit (Promega) that measured the degree of lucifer luminescence. In addition, it was confirmed whether the inhibitory effect on the NfkB signaling mechanism was enhanced compared to an experimental group treated with a tubulin inhibitor alone when 2 mM CaCl₂, 1 µM colchicine, 1 µM tapinarof, 1 µM fingolimod, and 1 µM tofacitinib were administered in combination with the tubulin inhibitor.

### [Experimental Example 4] Inhibitory effect of tubulin inhibitor on collagen reduction mechanism

Photoaging referred to a skin aging phenomenon that occurred when the skin was exposed to UV of the sun. The photoaging was a form of aging distinguished from biological aging (or senescence) due to a gradual decline in body functions and acted generally as the main cause of skin wrinkles, dryness, fibroelastosis, pigmentation, or the like.

The sun emitted two types of UV such as UV-A and UV-B, and the wavelengths were 320 to 400 nm for UV-A and 290 to 320 nm for UV-B. Among tissues damaged by the UV, collagen was a representative example. The collagen was the main ingredient of connective tissue and was mainly present in bones and skin and broadly classified into five types. Type 1 was the most abundant collagen found within the skin connective tissue, and Type 3 was the second abundant collagen.

Matrix Metalloproteinase (MMP) was a protease that degraded collagen as a major component of human connective tissue, and the expression level thereof was increased by UV. Accordingly, when the skin was exposed to UV, the expression of these Matrix Metalloproteinases (MMPs) increased to destroy collagen in the dermal layer of the skin and cause photoaging such as skin wrinkles and pigmentation.

The human skin cells NHEK were applied with the tubulin inhibitor or the control group, and then after 6 hours, irradiated with UB UV for 30 minutes. Intracellular RNA was extracted from skin cells using an RNeasy mini kit (Qiagen). After reverse transcription of the extracted RNA into cDNA using an ImProm-II^{™} Reverse Transcription kit (Promega), the expression levels of MMP-1, MMP-3, and MMP-9 present in skin cells were measured by a quantitative PCR (qPCR) method using the Real-Time PCR Detection System (Bio-Rad, CFX96).

### [Experimental Example 5] Confirmation of whitening function using measurement of tyrosinase activity

Tyrosinase activity was measured to determine whether the tubulin inhibitor of the present disclosure had a whitening function. Tyrosinase was an enzyme by which tyrosine was oxidized in the presence of oxygen to produce melanin, and the activity of tyrosinase was inhibited to inhibit melanin production. The tyrosine was oxidized by tyrosinase to turn orange, but when the tyrosinase activity was inhibited, the oxidation reaction of tyrosine through tyrosinase decreased, and then the tyrosine gradually became lighter to be transparent depending on the degree of tyrosinase activity inhibition. At this time, the decreased absorbance value was measured at 490 nm to evaluate the degree of tyrosinase inhibition.

### [Example 1] Filaggrin expression level and cell differentiation in human epidermal cell line treated with tubulin inhibitor

A normal human epidermal keratinocyte (NHEK) during culturing was treated with the tubulin inhibitor, and the expression level of filaggrin was measured through cell-based reporter assay and RT-PCR.

Each tubulin inhibitor was treated to NHEK at a concentration of 1.0 µM, and to compare filaggrin expression and cell differentiation, an equal dose of DMSO to the treated drug was treated to NHEK as a control, and 24 hours after administration, the expression level of filaggrin and the degree of cell differentiation were observed.

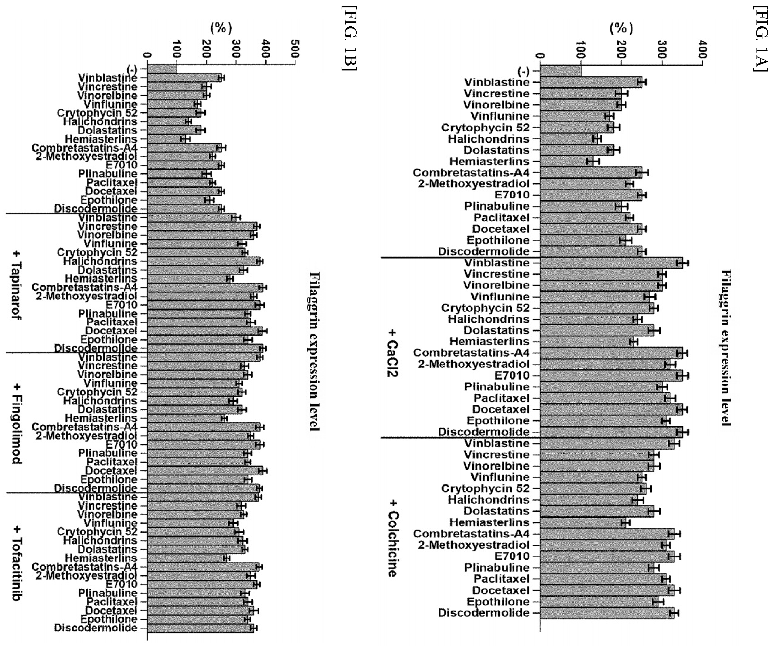

The results were shown in FIGS. 1 and 2.

As may be seen in FIG. 1A, the expression level of filaggrin increased in the tubulin inhibitor-treated group. In addition, as may be seen in FIG. 2A, it was confirmed that the degree of cell differentiation significantly increased in the tubulin inhibitor-treated group.

In addition, in order to confirm the effect of a combination with the tubulin inhibitor, when each tubulin inhibitor was treated in combination with 2 mM CaCl₂, 1 µM colchicine, 1 µM tapinarof, 1 µM fingolimod, and 1 µM tofacitinib, it was confirmed that the expression level of filaggrin (FIGS. 1A and 1B) and cell differentiation ability (FIGS. 2B and 2C) were increased compared to an experimental group treated with a tubulin inhibitor alone.

### [Example 2] Confirmation of inhibition of inflammatory mechanism by tubulin inhibitor

It was confirmed through a cell-based reporter assay that NF-κB activation was induced by treating a normal human epidermal keratinocyte (NHEK) with TNF-α, and then the NF-κB signaling was inhibited by treatment with the tubulin inhibitor at a concentration of 1.0 µM, and the results were shown in FIG. 3A.

As may be seen in FIG. 3A, treatment with the tubulin inhibitor according to the present disclosure inhibited NF-κB signaling compared to a control group treated only with TNF-α. A TPCA1 substance was used as a positive control, which was a well-known as a NF-κB signaling inhibitor.

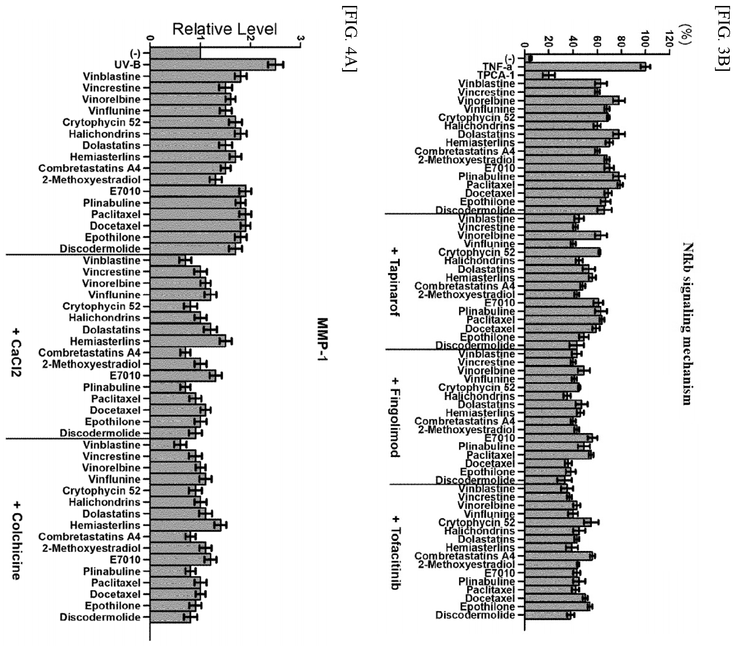

In addition, it was confirmed that the inhibitory effect on the NfkB signaling mechanism was enhanced compared to an experimental group treated with a tubulin inhibitor alone, when 2 mM CaCl₂, 1 µM colchicine, 1 µM tapinarof, 1 µM fingolimod, and 1 µM tofacitinib were administered in combination with the tubulin inhibitor (FIGS. 3A and 3B).

### [Example 3] Confirmation of inhibition of collagen reduction mechanism by tubulin inhibitor

Human NHEK cells were applied with a tubulin inhibitor, and irradiated with UV after 30 minutes to induce photoaging (especially, wrinkle alleviation). For comparison and verification of the tubulin inhibitor, an untreated negative control group and a positive control group irradiated only with UV-A were reflected in the experiment.

RNA was extracted from skin cells after application and UV irradiation experiment completion. After reverse transcription of the extracted RNA into cDNA, the amounts of MMP-1, MMP-3, and MMP-9 present in skin tissue were measured using a real-time PCR machine by a quantitative PCR (qPCR) method.

A photoaging inhibitory effect of the tubulin inhibitor was measured by changes in the expression levels of Matrix Metalloproteinase (MMP) proteins described above. That is, the expression levels of MMP proteins were measured in each of a control group irradiated only with UV, an experimental group irradiated with UV after applying a tubulin inhibitor, and a combined treatment group of a tubulin inhibitor and 2 mM CaCl₂, 1 µM colchicine, 1 µM tapinarof, 1 µM fingolimod, and 1 µM tofacitinib, to measure the degree of inhibiting the expression levels of MMP proteins by the application of the tubulin inhibitor.

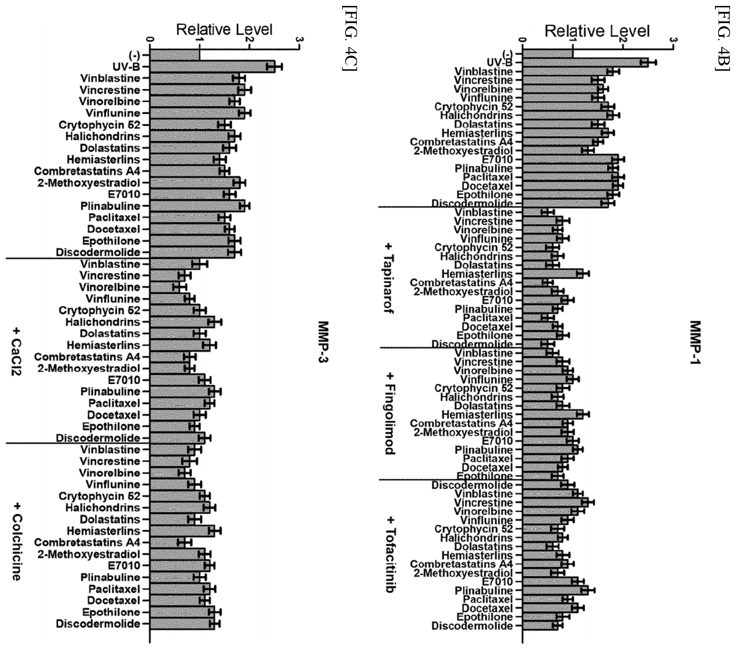

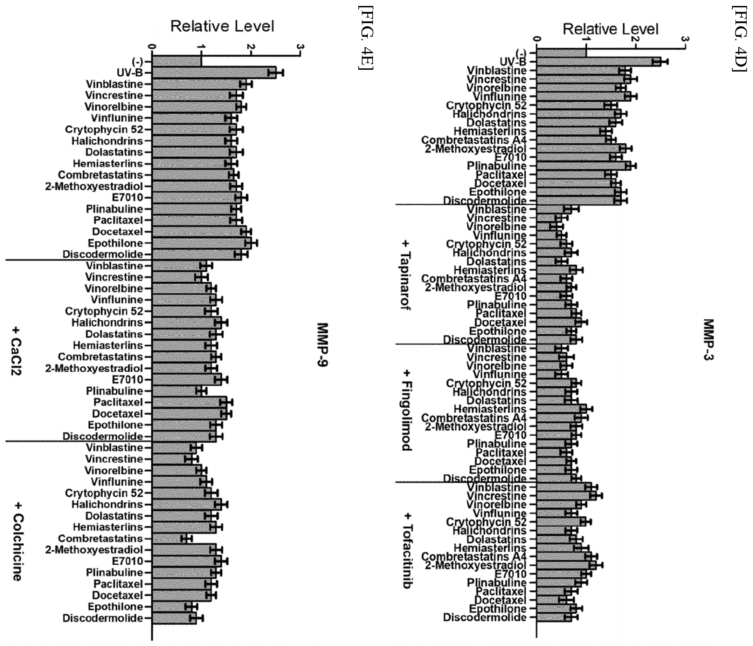

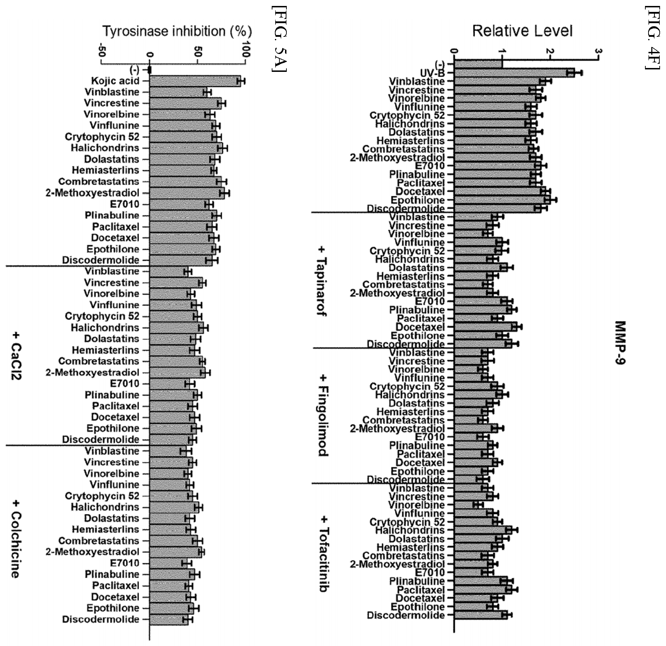

The results were illustrated in FIGS. 4A to 4F.

MMP-1 and MMP-9 were enzymes that promoted the degradation of Type 1 and Type 3 collagens, which were mainly expressed in skin tissue, and MMP-3 was a regulatory enzyme that degraded Type 1 collagen and regulated changes in the expression level of MMP-1, which were MMP proteins that had a very important effect on photoaging.

As may be seen in FIGS. 4A, 4C, and 4E, when the tubulin inhibitor was applied to the skin, it was confirmed that the expression levels of MMP-1, MMP-3, and MMP-9 were all reduced by about 50% compared to the control group irradiated only with UV. As such, it was confirmed that the expression levels of Matrix Metalloproteinases (MMP) proteins were inhibited by the application of the tubulin inhibitor, and overall, the inhibitory effect on photoaging caused by UV was confirmed. The effect of inhibiting the expression levels of MMP-1, MMP-3, and MMP-9, which were increased by UV-B irradiation, was measured according to treatment with each tubulin inhibitor.

In addition, it was confirmed that when 2 mM CaCl₂, 1 µM colchicine, 1 µM tapinarof, 1 µM fingolimod, and 1 µM tofacitinib were administered in combination with the tubulin inhibitor, the inhibitory effect on the expression levels of MMP-1, MMP-3, and MMP-9 was enhanced compared to an experimental group treated with a tubulin inhibitor alone (FIGS. 4A to 4F).

### [Example 4] Verification of effect of alleviating pigmentation by tubulin inhibitor

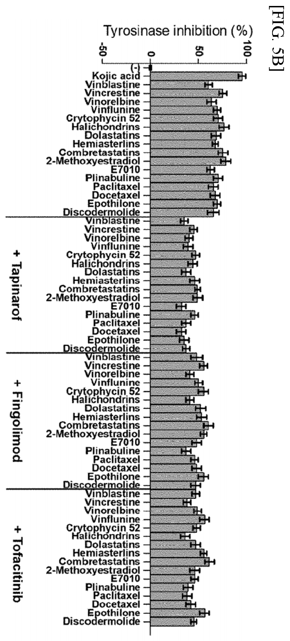

Through a tyrosinase activity inhibition experiment, a 2 mM L-tyrosine substrate was treated with each tubulin inhibitor at a concentration of 1.0 µM to measure the absorbance at 490 nm, and then added with tyrosinase and reacted at 37°C. After measuring the absorbance at 490 nm again, the absorbance value was calculated to determine the degree to inhibit the tyrosinase activity of each tubulin inhibitor. Kojic acid, known to inhibit the tyrosinase activity, was used as a comparative group. Through this, the degree to inhibit melanin production by each tubulin inhibitor may be determined. The results were illustrated in FIGS. 5A and 5B.

As shown in FIG. 5A, each tubulin inhibitor had the activity of inhibiting melanin production by inhibiting tyrosinase activity.

In addition, as shown in FIGS. 5A and 5B, it was confirmed that when 2 mM CaCl₂, 1 µM colchicine, 1 µM tapinarof, 1 µM fingolimod, and 1 µM tofacitinib were administered in combination with the tubulin inhibitor, the effect of inhibiting the tyrosinase activity was enhanced compared to an experimental group treated with the tubulin inhibitor alone.

## Claims

1. A pharmaceutical composition comprising a tubulin inhibitor as an active ingredient for preventing or treating skin diseases.

2. The pharmaceutical composition of claim 2, wherein the tubulin inhibitor is at least one selected from the group consisting of vinca alkaloid, taxane, cryptophycin 52, halichondrins, dolastatins, hemiasterlins, combretastatin-A4, 2-methoxyestradiol, E7010, plinabuline, epothilone, discodermolide, and pharmaceutically acceptable salts of the compounds.

3. The pharmaceutical composition of claim 2, wherein the vinca alkaloid is at least one selected from the group consisting of vinblastine, vincristine, vinorelbine, vinflunine, and pharmaceutically acceptable salts of the compounds.

4. The pharmaceutical composition of claim 2, wherein the taxane is at least one selected from the group consisting of paclitaxel, docetaxel, and pharmaceutically acceptable salts of the compounds.

5. The pharmaceutical composition of claim 1, wherein the skin disease is at least one selected from the group consisting of atopic dermatitis, skin thermal damage, pruritus, acne, psoriasis, allergic dermatitis, contact dermatitis, exfoliative dermatitis, seborrheic dermatitis, seborrheic scalpitis, lichen planus, rosacea, pigmentation disorder, hypermelanosis, erythema, wounds, ulcers, bedsores, lupus, skin wrinkle-related diseases, and skin diseases caused by photodamage.

6. The pharmaceutical composition of claim 5, wherein the skin wrinkle-related disease is at least one selected from the group consisting of elastic fibrosis, thinning of the skin, skin atrophy, reduction of collagen fibers and elastic fibers, loss of skin elasticity, dryness, wrinkle formation, and premature skin aging.

7. The pharmaceutical composition of claim 5, wherein the skin disease caused by the photodamage is at least one selected from the group consisting of lentigines, freckles, hypopigmentation, hyperpigmentation, acute or chronic photodamage caused by UV radiation, and photosensitization.

8. The pharmaceutical composition of claim 1, wherein the skin disease is at least one selected from the group consisting of squamous cell carcinoma, basal cell carcinoma, benign epithelial tumor, and radiation dermatitis.

9. The pharmaceutical composition of claim 1, wherein the skin disease is at least one selected from the group consisting of panniculitis, calluses, vitiligo, urticaria, folliculitis, sebaceous keratosis pilaris, eczema, corns, melasma, rashes, athlete's foot, spots, stretch marks, freckles, prickly heat, dry skin, chilblains, boils, pockmarks, scalpitis and psoriatic arthritis.

10. The pharmaceutical composition of claim 1, further comprising:
a compound selected from the group consisting of calcium, colchicine, tapinarof, fingolimod, and tofacitinib.

11. A quasi-drug composition comprising a tubulin inhibitor as an active ingredient for preventing or alleviating skin diseases.

12. A cosmetic composition comprising a tubulin inhibitor as an active ingredient for preventing or alleviating skin diseases.

13. A cosmetic composition comprising a tubulin inhibitor as an active ingredient for skin moisturization, whitening, wrinkle alleviation, acne relief, skin barrier strengthening, or skin keratinocyte differentiation.

14. A method for preventing or treating skin diseases, comprising administering a tubulin inhibitor in a therapeutically effective amount to a subject in need thereof.
